# EUROPEAN PATENT APPLICATION

(11) **EP 2 725 355 A1**
(43) Date of publication of application: **30.04.2014**
(21) Application number: 13189720.9
(22) Date of filing: 22.10.2013
(51) Int. Cl.: G01N 33/22

(54) **Method and device for measurement of the heating value of a gas stream**

(30) Priority: 25.10.2012 US 201213660011
(71) Applicant: Axetris AG, 6056 Kägiswil (CH)
(72) Inventor: Kinkade, Brian, Batavia, IL Illinois 60510 (US); Gregoria, Joe, McHenry, IL Illinois 60050 (US)
(74) Representative: Sebastian, Jens

(57) **Abstract**

A sample stream of the gas to be analyzed is fed into a combustor. The combustor mixes the gas with ambient air to an over-stoichiometric oxygen content and combusts the sample gas completely. A respective TDLS analyzer measures the CO₂ and/or H₂O concentration of the combustor's flue gas. In case that the sample gas contains relevant concentrations of CO₂ and/or H₂O, a second TDLS CO₂ and/or H₂O analyzer may be added which measures the CO₂ and/or or H₂O concentration upstream of the combustor. The measurement of methane and ethane or other hydrocarbon and mixtures thereof is also possible. The method and device allows the measurement of the heating value and/or the total organic carbon of a natural gas with high temporal resolution. The advantages of the invention are: Real-time determination of gas quality and energy value; real-time determination of Total Organic Carbons; if required, real-time measurement of the non-methane/non-ethane Total Organic Carbons; unattended, calibration- and service-free operation over extended periods of time and operation without consumables.

## Description

### Technical Field and Background of the Invention

The invention concerns a method and a device for measurement of the heating value of a gas stream.

Natural gas is a major fuel for a multitude of applications, ranging from large-scale power generation to kitchen equipment in households.

Its main constituent is methane (CH₄). Other constituents are higher-order hydrocarbons (ethane (C₂H₆) to hexane (C₆H₁₄)), and to some extent non-hydrocarbon gasses (oxygen (O₂), carbon dioxide (CO₂), water (H₂O) etc). The exact composition of any specific natural gas is dependent on the geographic location of the gas well, and on the subsequent treatment in the refinery.

The energy of a hydrocarbon, which will be released during its combustion is largely dependent on the number of carbon atoms per molecule, i.e. higher-order hydrocarbons will have higher energy. The energy in a gas is either expressed in British Thermal Units (BTU), where 1 BTU equals the energy required to raise the temperature of 1 British Pound of water for 1 degree Fahrenheit (as an Anglo-American unit) or in the SI-unit Joule (J), wherein 1 BTU is equal to 1055.05585262 J. Another common expression for the energy contained in a gas is the Wobbe Index, which relates the heating value of a gas to its specific gravity.

Due to varying compositions, the energy value for different resources of natural gas will vary geographically and/or over time. During any custody transfer, billing is based on the heating value. The heating value of any fuel gas is expressed e.g. in energy/volume of fuel (BTU per Standard Cubic Foot, BTU/SCF, or J/m³). According to the International Energy Agency the energy value varies from 33'320 kJ/m³ (Netherlands) to 42'000 J/m³ (Algeria). Thus it is essential for the buyer to know the exact heating value for the respective supply of natural gas. Other occasions where the knowledge of the energy value is important are various combustion processes, were the energy value serves as open loop control value for combustion control (i.e. flame temperature).

In the past, numerous measurement methods for the energy value of natural gas have been proposed. These methods can be classified into two groups:
In the first group, a sum parameter is measured, which is in relation to the energy value. Examples are the measurement of residual oxygen content in the flue gas of a combustor, or the heat developed by a pellistor element operating in a part of the gas stream, wherein combustible gas is burned at a catalytic active surface in a sufficiently rich oxygen atmosphere and the change in electrical resistance of a platinum wire is measured in a Wheatstone bridge arrangement.

In the second group of energy value analyzers, the specific composition of the natural gas is determined (by gas chromatography, NIR spectrometry, acoustic spectrometry or NMR spectrometry) and subsequently the energy value is calculated based on the specific gas composition.

Drawbacks for the energy value determination by sum parameters are the required precise, stoichiometric flow control of both natural gas and oxidation gas in order to control the combustion.

Drawbacks for energy value measurements by determination of the gas composition are expensive measurement devices, which need frequent calibration and service, and are often time consuming.

The mainstream measurement method today is gas chromatography (GC). GC yields very stable and precise results; however it is very slow, requires consumables, and is expensive and cumbersome. In practice, the supplier delivers a mix of different fields, pipelines, and storage facilities. The gas bill therefore leads to the so-called billing energy value for the accounting period. The energy value is nowadays usually measured with a so-called process gas chromatograph (PGC). This device is capable of analyzing in minutes up to 16 different constituents present in the gas, such as methane or propane. Because such PGC costs around 100,000 Euros, not each of the gas distribution companies has its own PGC installed at each entry point to the local gas network. When a large transport line supplies a number of gas suppliers, then one PGC suffices for the entire line, because the gas composition will not change downstream of the main supply line. The local gas distributor is thus based on the energy value measurement, which is made upstream in the transport line. At the transfer point usually only the delivered gas volume is measured. It is obvious, that that kind of measurement is not optimal.

### Summary of the Invention

The invention addresses the problem of proposing an improved method and device for measuring the energy value of a gas.

A method and a device as claimed solve that problem. Special features are claimed in the respective sub claims.

The invention provides the measurement of real-time values for the energy value and other gas composition parameters. It is less expensive than GC and does not require consumables. Further it allows automated operation without service or calibration over extended periods of time.

The measurement principle according to the invention is based on the fast measurement of at least one sum parameter downstream of a total combustion of the sample gas, and the concentration measurement of selected species upstream of the combustor.

In detail, according to the method of the invention, the method for measurement of the heating value of a gas stream, comprises providing a sample stream of the gas stream; total combustion of the sample gas in a combustor by injecting air into the combustor; selectively measuring the concentration of CO₂ or H₂O in the resulting flue gas with high temporal resolution, preferably by using a tuneable diode laser gas analyzer; calculating the heating value in view of the proportionality of the heating value and the CO₂ or H₂O concentration. The combustor completely burns a sample stream of the natural gas. In order to assure a complete combustion, ambient air is constantly injected into the combustor. This injection of air does not need to be regulated; however, it has to provide an over-stoichiometric amount of oxygen to the combustor in order to entirely combust the sample gas stream. A tuneable diode laser gas analyzer is able to measure the CO₂ concentration of the combustor's flue gas with sufficient selectivity and sufficient temporal resolution. However, other appropriate analysers might be used. During the combustion, every carbon atom of any hydrocarbon contained in the natural gas will be transformed into a molecule of CO₂. For alkanes, each atom of carbon will equally yield one molecule of H₂O, plus one molecule of H₂O per hydrocarbon molecule. The energy value for an alkane (CH₄ - C₆H₁₄, also called "C₁ - C₆") is roughly proportional to the number its carbon atoms. Under the assumptions that the upstream natural gas does not contain any CO₂, and that the volume of sample gas entering the combustor is constant over time, the CO₂ concentration of the combustor's flue gas will be proportional to the energy value of the sample gas. The deviation from the proportionality of the relation BTU content/order of the alkane increases with the number of carbon atoms to reach 22% for hexane (C₆H₁₄). However, as CH₄ is the major constituent and other alkanes only contribute with at maximum single digit concentration percentages, the overall linearity between energy value and CO₂ concentrations of the combustor's flue gas is sufficient for the majority of applications.

In case that the volume flow of sample gas entering the combustor cannot be held constant over time, according to a preferred embodiment of the invention, the total mass flow or volume flow of the sample gas fed into the combustor is measured and the measured gas concentration is related to the total mass flow or volume flow of the sample gas. Normalization of the CO₂ concentration in the combustor's flue gas by the mass flow or volume flow of sample gas entering the combustor will then yield a value which is proportional to the energy value of the sample gas, independently of the actual mass or volume flow.

According to another preferred embodiment of the invention a selective measurement of the concentration of certain gas constituents is performed, preferably concerning gas constituents selected from the group of CO₂, CO, H₂O, CH₄, C₂H₆, or the concentration of any other hydrocarbon and mixtures thereof of the sample gas upstream of the combustion. Many natural gases do contain a non-negligible concentration of CO₂. In such cases, the CO₂ concentration of the sample gas stream upstream of the combustor with sufficient selectivity and sufficient temporal resolution is measured. To determine the energy value, this CO₂ concentration upstream of the combustor is then deduced from the CO₂ concentration downstream of the combustor in the flue gas, and the resulting CO₂ concentration is proportional to the energy value as well as to the total organic carbon content (TOC). Also the H₂O concentration in the combustor's flue gas can be measured alone or additionally to the CO₂ concentration. In case that the natural gas already contains a non-negligible concentration of H₂O, its concentration may be measured upstream and downstream of the combustor and subsequently deduced from the H₂O concentration in the combustor's flue gas. As the resulting H₂O concentration is in direct relation to the concentration of carbon atoms in the natural gas, its interpretation will constitute a refinement, or a substitute, of the original energy value measurement by determination of the concentration of CO₂. Since in some locations, methane (CH₄) and ethane (C₂H₆) are not considered as volatile hydrocarbons by legislation. In such a case, according to the invention, measuring the respective concentrations of such gases upstream of the combustor is performed in order to obtain the legally relevant total organic carbons (TOC) content.

Preferably the method comprises measuring the total mass or volume flow of the sample gas before measuring one or more of the certain gas constituents, in order to avoid measurement inaccuracies.

Further, the method comprises selectively measuring of the gas constituents with high temporal resolution by one or more tuneable diode laser gas analyzers. This complies with the measurement of the fuel gas as mentioned above. An essential part of the invention are the incorporated means to precisely and selectively measure the concentrations of certain gases upstream and downstream of the combustor with a temporal resolution of a few seconds or better. Appropriate fast, selective gas sensors for the energy measurement according to the invention are for example tunable diode-laser spectrometry (TDLS) systems. Tunable diode laser gas analyzers make use of Vertical Cavity Surface Emitting Lasers, Distributed Feedback Lasers, Quantum Cascade Lasers or any other suitable laser diodes. Each TDLS system is tailored by choosing the wavelength of the near-infrared laser diode to match a specific gas absorption line with a selectivity of well below 1 nm, so that the corresponding gas will be measured with virtually no cross-sensitivity to other gases. The specifics of a TDLS system yield a highly linear signal for the gas concentration and a drift-free baseline, so that no calibration will be needed over periods of several years. A combined system to measure CO₂ and H₂O is estimated to have significantly lower cost than a GC installation, and the potential venue of diode lasers with large tuning range, covering several gas species, will contribute to lower the system cost. Preferably the tuneable diode laser gas analyzers are operated in the near (up to 3 µm wavelength) or mid (above 3 µm wavelength up to 16 µm) infrared spectrum. The single tuneable diode laser gas analyzer can contain a dedicated laser diode for each gas species to be measured or can contain at least one laser diode covering several gas species to be measured.

Advantageously, the sample gas stream is kept at defined temperatures throughout the embodiment in order to allow exact and defined measurements.

According to the invention, the heating value measurement device of a gas stream comprises in the gas stream a combustor with an air injector for injection air in the combustor, a selective gas analyzer for measuring the concentration of CO₂ or H₂O in the resulting flue gas with high temporal resolution, preferably a selective tunable diode laser analyzer, and electronic means for at least calculating measured values. Preferably, if necessary, a mass flow meter or volume flow meter is arranged upstream of the combustor between gas inlet and combustor.

In another preferred embodiment the combustor, the air injector and the gas analyser are all arranged in a first module. For certain applications (i.e. environmental protection applications), the user wants to measure the total organic carbons (TOC) content of a gas stream. As the TOC content is directly related to the number of carbon atoms in the hydrocarbons of the gas stream, the energy value-measuring device according to the invention can equally serve as a measurement device for TOC content.

For a more precise measurement the device according to the invention advantageously comprises a tuneable diode laser gas analyzer for selective measurement of the concentration of CO₂ or H₂O of the sample gas upstream of the combustor with high temporal resolution. Preferably a mass flow meter or volume flow meter can be arranged upstream the gas analyser between gas inlet and gas analyzer. Further the device can comprise means for measuring the concentration of CO₂ and/or H₂O of the flue gas of the sample gas downstream of the combustor with high temporal resolution, preferably additionally arranged in the first module.

In a further embodiment of the invention, the device comprises means for selective measurement of the concentration of certain gas constituents, preferably selected from the group of CO, CH₄, C₂H₆, or the concentration of any other hydrocarbon and mixtures thereof of the gas upstream of the combustor with high temporal resolution. Preferably the means to selectively measure a gas concentration of a gas constituent with high temporal resolution are a tuneable diode laser gas analyzer.

According to the invention means for selective measurement of the concentration of certain gas constituents, and, if used, a mass flow meter, all are arranged in a second module arranged upstream of the first module. This allows an optimal construction to use the different modules together, or to use the first module alone.

The device comprises at least in the first module electrical means for the control of the elements and determination of the measurement the results. The calculation and display of the final required result, such as energy value or TOC content corrected for specific hydrocarbons or any other value referring to the energy value or energy content of the sample gas (i.e. Wobbe Index), can be arranged in a separate unit.

The energy value measurement according to the invention has the following advantages:
- Real-time determination of gas quality and energy value;
- Real-time determination of Total Organic Carbons;
- If required, real-time measurement of the non-Methane/non-Ethane Total Organic Carbons;
- Unattended, calibration- and service-free operation over extended periods of time:
- Operation without consumables.

The same principle can also be employed for applications, where the amount of methane in natural, landfill or biogas gas, in respect to other organic carbons and contaminants, needs to be determined.

To the accomplishment of the foregoing and related ends, the invention, then, comprises the features hereinafter fully described and particularly pointed out in the claims. The following description and the annexed drawings set forth in detail certain illustrative embodiments of the invention. These embodiments are indicative, however, of but a few of the various ways in which the principles of the invention may be employed. Other objects, advantages, and novel features of the invention will become apparent from the following detailed description of the invention when considered in conjunction with the drawings.

### Brief Description of the Drawings

- Figure 1: a principle depiction of a first module, which also can be used for measuring the Total Organic Carbon content, and
- Figure 2: a principle depiction of the first module of Figure 1 together with a second module, arranged upstream the combustor in Figure 1.

### Detailed Description of the Drawings of the Invention

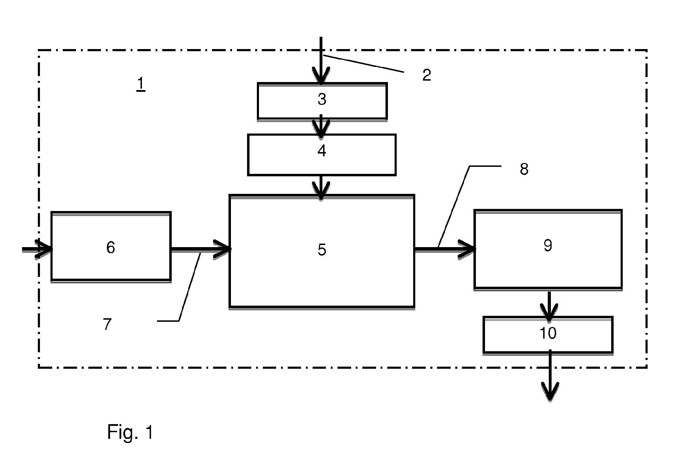

Figure 1 shows a basic energy value measurement device 1 according to the invention, which consists of a first module especially for measuring Total Organic Carbon (TOC). A sample stream 2 of a gas to be analyzed is led via a first flame arrestor 3 and facultative via a mass flow meter or a volume flow meter 4 into a combustor 5. The flame arrestor 3 and further mentioned flame resistors are necessary to ensure safety. In the combustor 5, the gas is mixed with air 7 injected by an air injector 6, and entirely combusted. A selective gas analyzer 9 for the concentration of CO₂ or alternatively H₂O of the gas then analyzes the flue gas 8 of the combustor 5, which will be proportional to the energy value of the gas. In case that a mass flow meter or volume flow meter 4 is installed between flame arrestor 3 and combustor 5, the CO₂ or H₂O concentration will be normalized by a mass or volume flow value delivered from the flow meter 4 in order to obtain a value, which is proportional to the energy value independently of the volume stream of the sample gas stream 2. The flue gas leaves the module 1 after having passed a second flame arrestor 10.

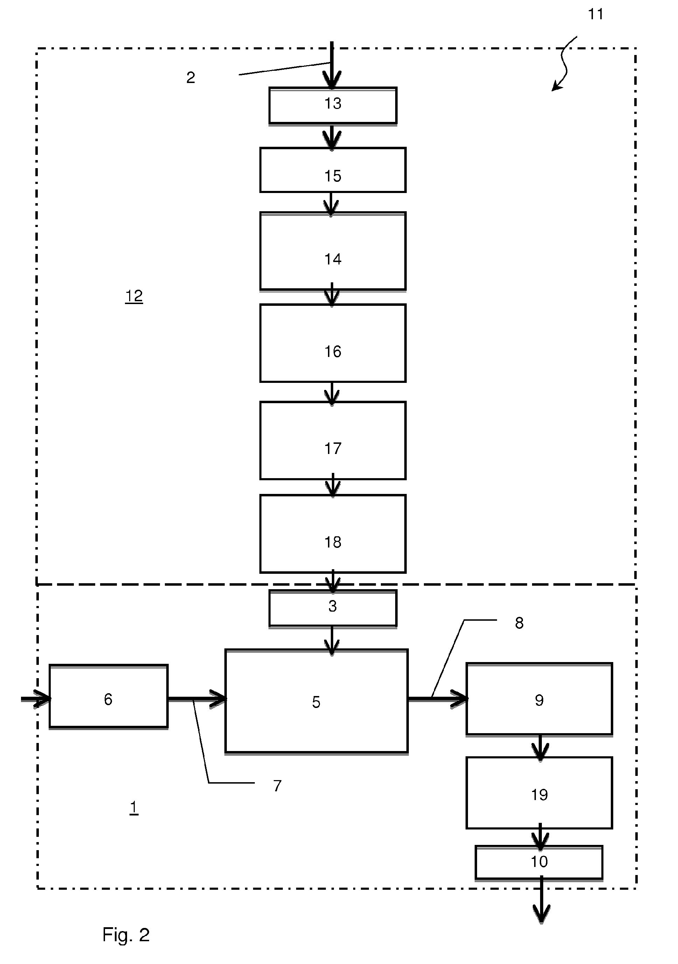

Figure 2 shows the layout of a general energy value measurement device 11 containing all options for analysing a gas in respect of energy value. The measurement device 11 is divided into the first module of Figure 1 with modifications and a second module 12 upstream of the first module 1. The basic version comprises in the second module 12 a third flame arrestor 13 and a further selective gas analyzer 14 for the concentration of CO₂ or alternatively H₂O of the gas. The basic version of the module 1 comprises in that arrangement no flow meter 4 as shown in Figure 1, but all the other elements of Figure 1 such as the first flame arrestor 3, combustor 5, air injector 6, selective gas analyser 9, and second flame arrestor 10.

For a more precise measurement in case of flow variations, the module 12 comprises a mass or volume flow meter 15 after the flame arrestor 13. The gas 2 enters the second module 12 through the third flame arrestor 13, passes the flow meter 15 and at least the selective gas analyser 14. For some applications, it is necessary to maintain a temperature of the gas within the second module above a certain temperature (i.e. 100°C) in order to avoid the condensation of water or other, temperature related issues.

The sample stream 2 of the natural gas leaves the second upstream module 12 to enter the first module 1 through the first flame arrestor 3 acting in that constellation for the sample stream 2 as a second flame arrestor. In the combustor 5 of the first module 1 the gas is mixed with the injected air and completely combusted. The selective gas analyzer 9 measures the concentration of CO₂ or H₂O in the flue gas 8 of the combustor 5. For some applications, it is necessary to maintain a temperature of the flue gas above a certain temperature (i.e. 100°C) in order to avoid the condensation of water or other, temperature related issues.. Having passed the gas analyzer 9, the flue gas 8 of the combustor 5 exits the first module 1 via the second flame arrestor 10 acting for the gas as a third flame arrestor. This arrangement of a second and a first module is necessary, if a CO₂ or H₂O gas concentration is present in the sample gas 2 and needs to be deduced from its concentration in the combustor's flue gas.

Facultative, the second module 12 may contain means to additionally selectively measure concentrations of various other gases according to the specific application. The different selective gas concentration measurements may be carried out subsequently in the first module, or in parallel.

Additionally the device 11 allows the measurement of both, CO₂ and H₂O gas concentration, with a gas analyser 16 in the second module 12 and a respective further gas analyser 19 in the first module 1. For example, gas analyser 14 and 9 measure CO₂ gas concentration and gas analyser 16 and 19 measure H₂O gas concentration. Optional the second module 12 comprises further several means 17 and 18 to selectively measure certain gas concentrations, e.g. methane (CH₄) with gas analyser 17 and ethane (C₂H₆) with gas analyser 18. The order of the different means of measurement 14, 16, 17, and 18 in the second module 12 is not relevant, as long as the total volume of the sample gas 2 entering the device 11 is measured by the optional flow meter 15.

The concentration values for the different gases are fed into an electronic circuitry (not shown), which relates the measurement values to energy value or TOC content.

For some applications, the sample stream 2 of natural gas in the energy value measurement device (upstream and/or downstream from the combustor 5) may be kept at defined temperature to avoid condensation or other temperature-related issues.

Appropriate fast, selective gas analyzers for the energy measurement according to the invention are for example tuneable diode-laser spectrometry (TDLS) systems. Tuneable diode laser gas analyzers are based on Vertical Cavity Surface Emitting Lasers, Distributed Feedback Lasers, Quantum Cascade Lasers or any other suitable laser diodes, which are appropriate to fulfil the requirements for the measurement.

## Claims

1. Method for measurement of the heating value of a gas stream, comprising
- providing a sample stream of the gas stream;
- total combustion of the sample gas in a combustor by injecting air in the combustor;
- selectively measuring of the concentration of CO₂ or H₂O in the resulting flue gas with high temporal resolution, preferably by using a tuneable diode laser gas analyzer;
- calculating the heating value or the total organic carbon content in view of the proportionality of the heating value or total organic content and the CO₂ or H₂O concentration.

2. Method of claim 1, comprising
measuring the total mass flow or volume flow of the sample gas fed into the combustor, and relating the measured gas concentration to the total mass flow of the sample gas.

3. Method of claim 1, comprising
selectively measuring the concentration of certain gas constituents, preferably selected from the group of CO, CO₂, H₂O, CH₄, C₂H₆, or the concentration of any other hydrocarbon and mixtures thereof of the sample gas upstream of the combustion with high temporal resolution and calculating the total organic carbon content after the combustion and measurement of the flue gas.

4. Method of claim 3, comprising
measuring the total mass flow of the sample gas before measuring one or more of the certain gas constituents.

5. Method of claim 3 or claim 4, comprising
selectively measuring of the gas constituents with high temporal resolution by one or more tuneable diode laser gas analyzers.

6. Method of anyone of the preceding claims, comprising keeping the sample gas stream at defined temperatures.

7. Method of anyone of the preceding claims, comprising operating the tuneable diode laser gas analyzers in the near or mid infrared spectrum.

8. Heating value measurement device of a gas stream, comprising in the gas stream a combustor with an air injector for injection air in the combustor, a selective gas analyzer for measuring the concentration of CO₂ or H₂O in the resulting flue gas with high temporal resolution, preferably a selective tuneable diode laser analyzer, and electronic means for at least calculating measured values.

9. Device according to claim 8, comprising
a mass flow meter or volume flow meter upstream of the combustor.

10. Device according to claim 8 or 9, wherein
the combustor, the air injector and the gas analyser are all arranged in a first module.

11. Device according to claim 8 comprising
a tuneable diode laser gas analyzer for selective measurement of the concentration of CO₂ or H₂O of the sample gas upstream of the combustor with high temporal resolution.

12. Device according to claim 11, comprising
a mass flow meter or volume flow meter between gas inlet and gas analyzer.

13. Device according to claim 11 or 12, comprising
means for selective measurement of the concentration of certain gas constituents, preferably selected from the group of CO, CH₄, C₂H₆, or the concentration of any other hydrocarbon and mixtures thereof of the sample gas upstream of the combustor with high temporal resolution.

14. Device according to any one of the preceding claims 11 to 13, where means for selective measurement of the concentration of certain gas constituents, and, if used, a mass flow meter, all are arranged in a second module arranged upstream of the first module.

15. Device according to claim 13, comprising
means for measuring the concentration of CO₂ and/or H₂O of the flue gas of the sample gas downstream of the combustor with high temporal resolution.

16. Device according to claim 15, wherein
said means are additionally arranged in the first module.

17. Device according to any one of the preceding claim 13, where the means to selectively measure a gas concentration of a gas constituent with high temporal resolution are a tuneable diode laser gas analyzer.
